# EUROPEAN PATENT APPLICATION

(11) **EP 1 323 702 A1**
(43) Date of publication of application: **02.07.2003**
(21) Application number: 01970216.6
(22) Date of filing: 26.09.2001
(51) Int. Cl.: C07C 51/58, C07C 59/58, C07C 69/708, C07C 51/04, C07C 59/315, B01F 17/44

(54) **PROCESS FOR PRODUCING ACYL FLUORIDE AND CARBOXYLIC ACID SALT**

(30) Priority: 27.09.2000 JP 2000294977; 01.03.2001 JP 2001057131
(71) Applicant: ASAHI GLASS COMPANY LTD., Tokyo 100-8405 (JP)
(72) Inventor: WATANABE, Kunio, c/o Asahi Glass Company, Limited, Yokohama-shi, Kanagawa 221-8755 (JP); OKAZOE, Takashi c/o Asahi Glass Company, Limited, Yokohama-shi, Kanagawa 221-8755 (JP); ITO, Masahiro, c/o Asahi Glass Company, Limited, Yokohama-shi, Kanagawa 221-8755 (JP); TATEMATSU, Shin, c/o Asahi Glass Company, Limited, Yokohama-shi, Kanagawa 221-8755 (JP); KAMIYA, Hiroki, Ichihara-shi, Chiba 290-8566 (JP); MUROTANI, Eisuke, c/o Asahi Glass Company, Limited, okohama-shi, Kanagawa 221-8755 (JP)
(74) Representative: Müller-Boré & Partner Patentanwälte
(86) International application number: JP0108368
(87) International publication number: WO02026686

(57) **Abstract**

A process for efficiently producing an acyl fluoride and a carboxylic acid salt while inhibiting side reactions.

R^{A}[OCH(CH₃)CH₂]ₙOH (1) and XCOR^{B} (R^{A} and R^{B} each is a group which upon fluorination becomes a perfluoroalkyl group, X is a halogen atom, and n is an integer of from 1 to 10) (2) are reacted to form an ester compound (3); the compound (3) is fluorinated in a liquid phase; and the ester bond of the fluorinated compound (4) is cleaved to obtain a perfluoroacyl fluoride (5). Further, this perfluoroacyl fluoride (5) is hydrolyzed and then reacted with ammonia or an alkali metal hydroxide to obtain a carboxylic acid salt (7).

## Description

### TECHNICAL FIELD

The present invention relates to a process for producing an acyl fluoride and a carboxylic acid salt, which are industrially useful. Further, the present invention relates to a novel compound which is useful as a fluororesin monomer or an emulsifier, or as a raw material therefor.

### BACKGROUND ART

As a method for fluorinating a C-H portion in a C-H containing compound to C-F, a method of employing cobalt trifluoride, a method of electrolyzing hydrogen fluoride in an electrolytic cell to carry out a fluorination reaction (an electrochemical fluorination method (hereinafter referred to as ECF method)), or a method of employing fluorine gas for fluorination, has been known. And, as the method of employing fluorine gas to carry out a fluorination reaction, a gas phase method and a liquid phase method are known.

As the liquid phase method, a method of reacting a compound containing hydrogen atoms with fluorine in a liquid phase to carry out fluorination, has been reported (U.S.P. 5,093,432). Further, a method is proposed wherein an ester containing hydrogen atoms is reacted with fluorine in a liquid phase to form a perfluoroester, which is then pyrolyzed in the presence of an inert solvent and a nucleophilic reagent containing no hydroxyl group, to obtain a perfluoroacyl fluoride (U.S.P. 5,466,877).

In the liquid phase method, in a usual case, a solvent capable of dissolving fluorine gas, is used as the liquid phase. However, the substrate for a fluorination reaction in conventional methods, usually has a low solubility in such a solvent, whereby there has been a problem such that the reaction will be a reaction in a suspension system which is disadvantageous to such a reaction, or a problem such that the production efficiency is poor since the reaction is carried out at an extremely low concentration. Further, the fluorination reaction will proceed also in a gas phase, whereby cleavage of C-C single bonds or C-O bonds will take place during the reaction, thus leading to a problem that by-products of various types will be formed, and a problem that the yield will be low.

It is an object of the present invention to provide a process for efficiently producing an acyl fluoride and a carboxylic acid salt, which are useful as raw materials for a fluorinated monomer or an emulsifier, while inhibiting side reactions.

### DISCLOSURE OF THE INVENTION

The present invention provides the following invention whereby the above object can be accomplished.
1. A process for producing a compound of the following formula (5) (hereinafter, a compound of the formula (5) will be represented as the compound (5), and compounds of other formulae will be likewise represented), or the following compound (5) and the following compound (6), which comprises reacting the following compound (1) and the following compound (2) to form of the following compound (3), fluorinating in a liquid phase the compound (3) thus formed, to obtain the following compound (4), and then cleaving an ester bond in the compound (4):
   (1) R^{A} [OCH(CH₃)CH₂] ₙ OH
   (2) XCOR^{B}
   (3) R^{A} [OCH(CH₃)CH₂] ₙ OCOR^{B}
   (4) R^{AF} [OCF(CF₃)CF₂] ₙ OCOR^{BF}
   (5) R^{AF} [OCF(CF₃)CF₂] ₙ₋₁ OCF(CF₃)COF
   (6) FCOR^{BF}
   wherein R^{A} is a group which becomes R^{AF} upon fluorination, R^{AF} is a perfluoroalkyl group, R^{B} is the same monovalent organic group as R^{BF} or a monovalent organic group which becomes R^{BF} upon fluorination, R^{BF} is a monovalent organic group, X is a halogen atom, and n is an integer of from 1 to 10.
2. The above process, wherein R^{BF} is R^{AF}[OCF(CF₃)CF₂]ₙ₋₁OCF(CF₃)-, wherein n is as defined above.
3. The above process, wherein n is 2.
4. A process for producing the following compound (7), which comprises hydrolyzing the compound (5) obtained by the above process, followed by a reaction with ammonia or an alkali metal hydroxide:
   (7) R^{AF} [OCF (CF₃) CF₂] ₙ₋₁ OCF (CF₃) COO⁻M⁺
   wherein R^{AF} and n are as defined above, M⁺ is NH₄⁺ or an alkali metal cation.
5. The following compound (4A): 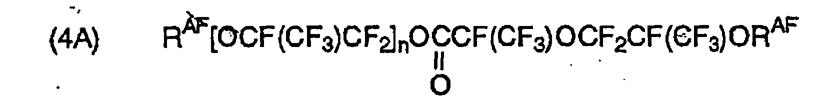 wherein R^{AF} is a perfluoroalkyl group, and n is an integer of from 1 to 10.
6. The following compound (7A):
   (7A) R^{AF1} [OCF (CF₃) CF₂]ₙ₋₁OCF (CF₃) COO⁻M⁺
   wherein R^{AF1} is a C₄₋₁₀ perfluoroalkyl group, n is an integer of from 1 to 10, and M⁺ is NH₄⁺ or an alkali metal cation.
7. The above compound (7A), wherein n is 1.
8. The above compound (7A), wherein R^{AF1} is C₆F₁₃-.
9. An emulsifier comprising the above compound (7A).

### BEST MODE FOR CARRYING OUT THE INVENTION

In this specification, a compound of the formula (1) will be represented as the compound (1). Compounds of other formulae will be likewise represented.

R^{A} in the compound (1) is a group which becomes R^{AF} upon fluorination, i.e. a group which becomes a perfluoroalkyl group.

In the present invention, the structure of R^{A} in the compound (1) will determine the basic carbon skeleton of R^{AF} in the compound (5). Accordingly, the liquid phase fluorination reaction is carried out by using the compound (1) having a group (R^{A}) which corresponds to such R^{AF}. Such group (R^{A}) may be a partially fluorinated alkyl group having some of fluorine atoms in the desired perfluoroalkyl group (R^{AF}) substituted by hydrogen atoms, an alkyl group having all of fluorine atoms in the desired R^{AF} substituted by hydrogen atoms, or a group having a carbon-carbon single bond in R^{AF}, or in said partially fluorinated alkyl group or said alkyl group, substituted by a carbon-carbon unsaturated bond (such as a double bond or a triple bond). The structure of R^{A} may be optionally changed taking into consideration the availability of the compound (1) or the economical efficiency. Among them, as R^{A}, for such a reason that various structures are generally readily available inexpensively or readily synthesized, R^{A} is preferably an alkyl group, more preferably a C₁₋₁₆ alkyl group, particularly preferably a C₁₋₈ alkyl group. Further, n is an integer of from 1 to 10. From the viewpoint of the usefulness of the compound, n is preferably 1 or 2, and particularly preferably, n is 2.

The structure of the alkyl group may be a linear structure, a branched structure, a cyclic structure or a structure partially having a cyclic structure. The alkyl group having a linear structure may be H(CH₂)ₘ- (wherein m is an integer of from 1 to 16). The alkyl group having a branched structure may, for example, be an isopropyl group, an isobutyl group, a sec-butyl group or a tert-butyl group. The alkyl group having a cyclic structure (i.e. the cycloalkyl group) may, for example, be a cyclopropyl group, a cyclobutyl group, a cyclopentyl group, a cyclohexyl group or a group having an alkyl group of a linear structure or a branched structure bonded to a carbon atom constituting the ring of such a group. The alkyl group partially having a cyclic structure may be an alkyl group of a linear structure having a cycloalkyl group substituted thereon, or an alkyl group of a branched structure having a cycloalkyl group substituted thereon, and a cyclohexyl methyl group, a cyclohexyl methyl group, a cyclohexyl ethyl group or the like, is preferred. R^{A} is preferably an alkyl group, more preferably an alkyl group of a linear structure. Further preferably, R^{A} is a C₄₋₁₀ alkyl group, particularly preferably H(CH₂)ₘ- (m is an integer of from 4 to 10), especially preferably such a group wherein m is from 1 to 6.

The following compounds may be mentioned as specific examples of the compound (1). Here, m and n are as defined above, and Cy represents a cyclohexyl group.

H (CH₂) ₘ [OCH (CH₃) CH₂] ₙ OH,

(CH₃) ₂ CH [OCH (CH₃) CH₂] ₙ OH,

(CH₃) ₂ CHCH₂ [OCH (CH₃) CH₂] ₙ OH,

(CH₃) ₃ C [OCH (CH₃) CH₂] ₙ OH,

and

Cy [OCH (CH₃) CH₂] ₙ OH.

The compound (1) is a compound which is readily available or which can easily be synthesized by a known method. For example, 2-alkoxyalcohols can easily be synthesized by methods disclosed in J. Am. Chem. Soc., 49, 1080 (1927), Bull. Soc. Chim. Fr., 1813 (1960), Can. J. Chem., 43, 1030 (1965), Synthesis, 280 (1981), etc.

In the present invention, the compound (1) is reacted with the compound (2). The halogen atom represented by X in the compound (2) is a fluorine atom, a chlorine atom, a bromine atom or an iodine atom, preferably a fluorine atom, a chlorine atom or a bromine atom. Further, in a case where the after-mentioned continuous process is carried out, it is preferably a fluorine atom. -CH₂OH in the compound (1) will react with XCO- (X is a halogen atom) in the compound (2) to form a bond (-CH₂OCO-).

R^{B} in the compound (2) is the same monovalent organic group as R^{BF} or a group which becomes R^{BF} upon fluorination.

In a case where R^{B} is the same monovalent organic group as R^{BF}, R^{B} is a monovalent organic group which undergoes no change by the fluorination reaction. Such a monovalent organic group is a monovalent organic group having no structure which can be fluorinated. For example, in a case where R^{B} is a perhalogenated monovalent organic group such as a perfluoroalkyl group, such a group is a monovalent organic group which undergoes no change by a fluorination reaction.

The compound (2) in the present invention is preferably a compound wherein R^{B} is a monovalent organic group containing fluorine atoms, for such a reason that in the subsequent liquid phase fluorination reaction, the compound (3) will be readily soluble in the liquid phase. Further, from the viewpoint of easy industrial production or availability of the compound, R^{B} in the compound (2) is preferably a perfluorinated monovalent organic group, particularly preferably a perfluoroalkyl group having substantially all hydrogen atoms in an alkyl group substituted by fluorine atoms, or a perfluoro(etheric oxygen atom-containing alkyl) group containing an etheric oxygen atom and having substantially all hydrogen atoms in an alkyl group substituted by fluorine atoms.

Preferred examples of the perfluorinated monovalent organic group include a C₁₋₂₀ perfluoroalkyl group and a C₁₋₂₀ perfluoro(etheric oxygen atom-containing alkyl) group.

The C₁₋₂₀ perfluoroalkyl group is a group having all hydrogen atoms in a C₁₋₂₀ alkyl group substituted by fluorine atoms, and a C₁₋₁₀ perfluoroalkyl group is preferred. Specific examples of the perfluoroalkyl group may be groups shown in the specific examples of the compound (4) given hereinafter. In a case where the perfluoroalkyl group is of a linear structure, -CF₃, -CF₂CF₃, -CF₂CF₂CF₃, and -CF₂CF₂CF₂CF₃ are, for example preferred. In a case where the perfluoroalkyl group is of a branched structure, -CF(CF₃)₂, -CF₂CF(CF₃)₂, -CF(CF₃)CF₂CF₃ and -C(CF₃)₃ are, for example, preferred.

The C₁₋₂₀ perfluoro(etheric oxygen atom-containing alkyl) group is a group having all hydrogen atoms in the above mentioned etheric oxygen atom-containing alkyl group substituted by fluorine atoms, and a C₁₋₁₀ perfluoro(etheric oxygen atom-containing alkyl) group is preferred. The perfluoro(etheric oxygen atom-containing alkyl) group is preferably a perfluoroalkoxyl group, perfluoro(alkoxyalkyl) group or a perfluoro(alkoxyalkoxyalkyl) group.

Further, as the compound (2), it is preferred to use the compound (6) which will be described hereinafter, as the compound (2), since it is thereby possible to carry out a continuous production efficiently. Namely, R^{B} is preferably the same monovalent organic group as R^{BF}, specifically the group described as a preferred group for R^{BF}.

The following compounds may be mentioned as specific examples of the compound (2). Here, k is an integer of from 1 to 20, preferably an integer of from 1 to 10, p is an integer of from 0 to 9, preferably 1, q is an integer of from 1 to 16, preferably an integer from 1 to 8, and Cy^{F} represents a perfluorocyclohexyl group.

FCO(CF₂)ₖF ,

FCOCF(CF₃)O [CF₂CF (CF₃) O] ₚ (CF₂) _{q} F ,

FCOCF(CF₃)O [CF₂CF (CF₃) O] ₚ CF (CF₃) ₂ ,

FCOCF(CF₃)O [CF₂CF (CF₃) O] ₚ CF₂CF (CF₃) ₂ ,

FCOCF(CF₃)O [CF₂CF (CF₃) O] ₚ C(CF₃)₃ ,

and

FCOCF(CF₃)O [CF₂CF (CF₃) O] ₚ Cy^{F} .

The compound (2) may be a commercial product or the compound (5) which will be formed by the process of the present invention, which will be described hereinafter. Further, the compound (2) is a compound which is readily available, for example, as an intermediate for a perfluoro(alkyl vinyl ether).

Further, for the compound (2), it is preferred to select R^{B} so that the compound (5) and the compound (6) to be formed by the cleavage reaction of the ester bond, which will be described hereinafter, will be the same compounds. Namely, R^{B} is preferably R^{AF}[OCF(CF₃)CF₂]ₙ₋₁OCF(CF₃)- or a group which becomes such a group upon a fluorination reaction. Particularly preferred is such a group wherein n is 2 (i.e. R^{AF}OCF(CF₃)CF₂OCF(CF₃)-), since it is thereby possible to obtain an emulsifier having a particularly excellent performance.

The reaction of the compound (1) and the compound (2), can be carried out under reaction conditions of a known esterification reaction. Such a reaction may be carried out in the presence of a solvent (hereinafter referred to as an esterification solvent), but it is preferably carried out in the absence of an esterification solvent from the viewpoint of the volume efficiency. It is particularly preferred to carry out the reaction in the presence of an excess amount of the compound (2) relative to the compound (1). In a case where an esterification solvent is to be used, dichloromethane, chloroform, triethylamine or a mixed solvent of triethylamine and tetrahydrofuran, is preferred. The amount of the esterification solvent to be used, is preferably from 50 to 500 mass%, based on the total amount of the compound (1) and the compound (2).

By the reaction of the compound (1) and the compound (2), an acid represented by HX will be formed. In a case where a compound wherein X is a fluorine atom, is used as the compound (2), HF will be formed. Accordingly, as a HF scavenger, an alkali metal fluoride (preferably NaF or KF) or a trialkylamine may be present in the reaction system. It is advisable to use a HF scavenger, in a case where the compound (1) or the compound (2) is a compound instable to an acid. Further, in a case where no HF scavenger is used, it is preferred to carry out the reaction at a reaction temperature where HF can be vaporized, and HF is discharged out of the reaction system, as carried by a nitrogen stream. It is preferred that the HF scavenger is from 1 to 10 times by mol to the compound (2).

With respect to the amounts of the compound (1) and the compound (2) to be used for the reaction, the amount of the compound (2) to the compound (1) is preferably from 0.75 to 5 times by mol, more preferably from 1 to 2.5 times by mol, particularly preferably from 1.5 to 2.5 times by mol.

The temperature for the reaction of the compound (1) and the compound (2) is, in a usual case, preferably at least -50°C and at most +100°C or at most the boiling point of the solvent.

Further, the reaction time for the reaction may be optionally changed depending upon the supply rates of the feed materials and the amounts of the compounds to be used for the reaction. The pressure for the reaction (gauge pressure, the same applies hereinafter) is preferably from 0 to 2 MPa.

By the reaction of the compound (1) and the compound (2), the compound (3) will be formed. The crude product containing the compound formed by the reaction of the compound (1) and the compound (2), may be purified depending upon the particular purpose, or may be used for the next reaction, etc. as it is. However, it is advisable to purify the crude product, so that the fluorination reaction in the next step can be carried out constantly.

The method for purifying the crude product may, for example, be a method of distilling the crude product as it is, a method of treating the crude product with a dilute alkaline aqueous solution, followed by liquid separation, a method of extracting the crude product with a suitable organic solvent, followed by distillation, or silica gel column chromatography.

R^{A} in the compound (3) is the same group as R^{A} in the compound (1). R^{B} is the same group as R^{B} in the compound (2).

With respect to the fluorine content in the compound (3) (the proportion of the total amount of fluorine atoms to the molecular weight of the compound), in order to improve the solubility in the liquid phase at the time of the fluorination reaction, the lower limit of the fluorine content is preferably adjusted to be 30 mass%, particularly preferably 50 mass%, by adjusting the structures of R^{A} and R^{B} in the compound (3). Further, the upper limit of the fluorine content is preferably 86 mass%, particularly preferably 70 mass%. The molecular weight of the compound (3) is preferably from 200 to 1000, from such a viewpoint that the fluorination reaction in a liquid phase can be carried out smoothly. If the molecular weight is too small, the compound (3) tends to be readily vaporized, whereby a decomposition reaction is likely to take place in the gas phase during the fluorination reaction. On the other hand, if the molecular weight is too large, purification of the compound (3) is likely to be difficult.

The following compounds may be mentioned as specific examples of the compound (3). Some of these compounds are novel compounds.

In the following formulae, m, n, k, p and q are as defined above.

(3A) H (CH₂) ₘ [OCH (CH₃) CH₂] ₙ OCO (CF₂) ₖ F ,

(3B) H (CH₂) ₘ [OCH (CH₃) CH₂] ₙ OCOCF (CF₃) O [CF ₂ CF (CF₃) O] ₚ (CF₂) _{q} F ,

(3C) (CH₃) ₂ CH [OCH (CH₃) CH₂] ₙ OCOCF (CF₃) O [C F₂CF (CF₃) O] ₚ CF (CF₃) ₂ ,

and

(3D) (CH₃) ₃ C [OCH (CH₃) CH₂] ₙ OCOCF (CF₃) O [CF ₂ CF (CF₃) O] ₚ C (CF₃) ₃ .

Among compounds (3), the compound (3B) is preferred from the viewpoint such that a continuous production process can be carried out, and the product is useful. Particularly preferred is the following compound (3B-1) wherein m and q have the same value, and p has the same value as (n-1).

(3B-1) H (CH₂) ₘ [OCH (CH₃) CH₂] ₙ OCOCF (CF₃) O [CF₂CF (CF₃) O] ₙ₋₁ (CF₂) ₘ F

Then, in the present invention, the compound (3) is fluorinated in a liquid phase to form the compound (4).

R^{AF} in the compound (4) is a perfluoroalkyl group, preferably a C₁₋₁₆ perfluoroalkyl group, particularly preferably a C₁₋₈ perfluoroalkyl group, especially preferably F(CF₂)ₘ- (m is as defined above), further preferably a CF₃CF₂CF₂- group.

R^{BF} in the compound (4) is a monovalent organic group. When R^{B} is a monovalent organic group having no structure to be fluorinated, R^{BF} is the same group as R^{B}, and when R^{B} is a monovalent organic group having a structure to be fluorinated (such as a hydrogen atom), it is a group having substantially all of such a structure fluorinated (i.e. a perfluorinated group).

R^{BF} is preferably a perfluorinated monovalent organic group, more preferably a perfluoroalkyl group or a perfluoro(etheric oxygen atom-containing alkyl) group, particularly preferably R^{AF}[OCF(CF₃)CF₂]ₙ₋₁ OCF (CF₃)-(wherein n is as defined above). When R^{BF} is such a group, the compounds (5) and (6) to be formed by the subsequent cleavage reaction of the ester bond, will be the same, whereby post treatment can be simplified, such being more preferred.

The following compounds may be mentioned as specific examples of the compound (4).

In the following formulae, m, n, k, p and q are as defined above.

(4A) F (CF₂) ₘ [OCF (CF₃) CF₂] ₙ OCO (CF₂) ₖ F

(4B) F (CF₂) ₙ [OCF (CF₃) CF₂] ₙ OCOCF (CF₃) O [CF ₂ CF (CF₃) O] ₚ (CF₂) _{q} F

(4C) (CF₃) ₂ CF [OCF (CF₃) CF₂] ₙ OCOCF (CF₃) O [C F₂CF (CF₃) O] ₚ CF (CF₃) ₂

(4D) (CF₃) ₃ C [OCF (CF₃) CF₂] ₙ OCOCF (CF₃) O [CF ₂ CF (CF₃) O] ₚ C (CF₃) ₃

Among the above compounds (4), compound (4B), compound (4C) and compound (4D) are preferred in that a continuous production method can be carried out, and the product is useful. Particularly preferred is the following compound (4B-1), wherein m and q in the compound (4B) have the same value, and p has the same value as (n-1).

(4B-1) F (CF₂) ₘ [OCF (CF₃) CF₂] ₙ OCOCF (CF₃) O [CF₂CF (CF₃) O] ₙ₋₁ (CF₂) ₘ F

In the present invention, the compound (3) is fluorinated in a liquid phase by means of fluorine (elemental fluorine) for perfluorination. The compound (3) may be fluorinated by a gas-solid reaction at a high temperature by means of cobalt fluoride. However, by such a method, isomerization or cleavage of the C-O bond is likely to take place, thus leading to a problem that by-products of many types will be formed. Further, the compound (3) may be fluorinated by the ECF method, but by such a method, cleavage of the C-O bond is likely to take place, thus leading to a problem that the product of the fluorination reaction cannot be obtained in good yield. Thus, the liquid phase fluorination method is an extremely advantageous method for industrial operation, since the yield is high and the operation of the reaction is easy as compared with the method of employing cobalt trifluoride or the ECF method.

As the fluorine to be used for the liquid phase fluorination reaction of the present invention, fluorine gas may be used as it is, or fluorine gas diluted with an inert gas, may be employed. As such an inert gas, nitrogen gas or helium gas is preferred, and from an economical reason, nitrogen gas is particularly preferred. The amount of fluorine gas in the nitrogen gas is not particularly limited, and it is preferably at least 10 vol%, from the viewpoint of the efficiency, particularly preferably at least 20 vol%.

The liquid phase in the fluorination reaction may be the substrate or the product of the fluorination reaction, but it is usually preferred to employ a solvent. Such a solvent (hereinafter referred to as a fluorination solvent) is preferably a solvent which essentially contains a C-F bond without containing a C-H bond. It is further preferably a perfluoroalkane or an organic solvent obtained by perfluorinating a known organic solvent having at least one type of atom selected from a chlorine atom, a nitrogen atom and an oxygen atom in its structure. Further, it is preferred to use a solvent wherein the solubility of the compound (3) is high, as the fluorination solvent. It is particularly preferred to employ a solvent capable of dissolving at least 1 mass% of the compound (3), especially a solvent capable of dissolving at least 5 mass% thereof.

Examples of the fluorination solvent include the compound (2), the compound (4), the compound (5), the compound (6), a perfluoroalkane (such as FC-72, trade name), a perfluoroether (such as FC-75 or FC-77, trade name), a perfluoropolyether (such as KRYTOX, FOMBLIN, GALDEN or DEMNUM, trade name), a chlorofluorocarbon (Flon Lube, trade name), a chlorofluoropolyether, a perfluoroalkylamine (such as a perfluorotrialkylamine), and an inert fluid (FRORINART, trade name). In a case where the compound (4), the compound (5) or the compound (6) is used as the liquid phase for the fluorination reaction, there is a merit such that post treatment after the reaction will be easy. Especially when R^{B} is R^{AF}[OCF(CF₃)CF₂]ₙ₋₁OCF(CF₃)-, it is extremely efficient and preferred to employ such a compound as the fluorination solvent. The amount of the fluorination solvent is preferably at least 5 times by mass, particularly preferably from 10 to 100 times by mass, based on the compound (3).

As the reaction system for the fluorination reaction, a batch system or a continuous system is preferred. Especially from the viewpoint of the yield and selectivity in the reaction, the following method (2) is preferred. Further, fluorine gas is preferably used as diluted with an inert gas such as nitrogen gas, whether the reaction is carried out by a batch system or by a continuous system.

### Method (1)

A method wherein the compound (3) and the fluorination solvent are charged into a reactor, and stirring is initiated, and the reaction is carried out while continuously supplying fluorine gas into the fluorination solvent at a prescribed reaction temperature under a prescribed reaction pressure.

### Method (2)

A method wherein the fluorination solvent is charged into the reactor, and stirring is initiated, and then the compound (3) and the fluorination solvent are continuously and simultaneously supplied in a prescribed molar ratio at a prescribed reaction temperature under a prescribed reaction pressure.

In the method (2), when the compound (3) is supplied, it may or may not be diluted with the fluorination solvent. Further, in the method (2), when the compound (3) is diluted with the solvent, the amount of the fluorination solvent to the compound (3) is preferably at least 5 times by mass, particularly preferably at least 10 times by mass.

With respect to the amount of fluorine to be used for the fluorination reaction, it is preferred to carry out the reaction in such a state that fluorine is present so that the amount of fluorine is always in an excess equivalent to hydrogen atoms in the compound (3) whether the reaction is carried out by a batch system or by a continuous system. Particularly, from the viewpoint of the selectivity, it is preferred that fluorine is present so that it will be at least 1.5 times by equivalent (i.e. at least 1.5 times by mol). It is preferred that the amount of fluorine is always kept to be in an excess amount from the initiation to the completion of the fluorination reaction.

The reaction temperature for the fluorination reaction is usually at least -60°C and at most the boiling point of the compound (3). From the viewpoint of the yield in reaction, the selectivity and easy industrial operation, it is particularly preferably from -50°C to +100°C, especially preferably from -20°C to +50°C. The reaction pressure for the fluorination reaction is not particularly limited, and it is particularly preferably from atmospheric pressure to 2 MPa from the viewpoint of the yield in reaction, the selectivity and efficient industrial operation.

Further, in order to let the fluorination reaction proceed efficiently, it is preferred to add a C-H bond containing compound to the reaction system or to carry out ultraviolet irradiation at a later stage of the reaction. For example, in a batch system reaction, it is preferred to add a C-H bond containing compound to the reaction system or to carry out ultraviolet irradiation at a later stage of the fluorination reaction. In a continuous system reaction, it is preferred to supply a C-H bond containing compound or to irradiate ultraviolet rays. It is thereby possible to efficiently fluorinate the compound (3) present in the reaction system and to improve the conversion remarkably.

The C-H bond containing compound may preferably be an organic compound other than the compound (3), particularly preferably an organic hydrocarbon, especially preferably benzene, toluene or the like. The amount of such a C-H bond containing compound is preferably from 0.1 to 10 mol%, particularly preferably from 0.1 to 5 mol%, based on the hydrogen atoms in the compound (3).

Further, the C-H bond containing compound is preferably added into a reaction system where fluorine is present. Further, in a case where a C-H bond containing compound is added, it is preferred to pressurize the reaction system. The pressure for the pressurizing is preferably from 0.01 to 5 MPa.

In the reaction to fluorinate the compound (3) in a liquid phase, a reaction to substitute a hydrogen atom by a fluorine atom will take place, whereby HF will be formed as a by-product. To remove HF formed as a by-product, it is preferred to let a HF scavenger be present in the reaction system or to let the discharge gas contact with a HF scavenger at the gas outlet of the reactor. As such a HF scavenger, the same one as described above may be employed, and NaF is preferred.

In a case where a HF scavenger is permitted to be present in the reaction system, the amount is preferably from 1 to 20 times by mol, particularly preferably from 1 to 5 times by mol, to the total amount of hydrogen atoms present in the compound (3). In a case where a HF scavenger is set at the gas outlet of the reactor, it is preferred that (a) a cooler (preferably maintained at a temperature of from 10°C to room temperature, particularly preferably at about 20°C), (b) a layer packed with NaF pellets and (c) a cooler (preferably maintained at a temperature of from -78°C to +10°C, more preferably from -30°C to 0°C) are set in series in the order of (a)-(b)-(c). Further, a liquid returning line to return a condensed liquid from the cooler of (c) to the reactor, may be installed.

The crude product containing the compound (4) obtained by the fluorination reaction may be used as it is for the next step, or may be purified to one having a high purity. The purification method may, for example, be a method of distilling the crude product as it is under atmospheric pressure or reduced pressure.

In the present invention, the ester bond in the compound (4) will further be cleaved.

The cleavage reaction of an ester bond is a reaction to cleave -CF₂OCO- in the compound (4) to form -COF. The method and conditions of the reaction may suitably be changed depending upon the structure of the compound (4). The reaction to cleave the ester bond is preferably carried out by heating or by reacting it with a nucleophilic agent or an electrophilic agent in a liquid phase.

As the reaction system for the cleavage reaction of the ester bond by heating, a gas phase reaction or a liquid phase reaction may be mentioned, and it is preferably selected depending upon the boiling point and the stability of the compound (4). For example, it is preferred that in the case of a compound (4) having a low boiling point, a gas phase reaction is carried out, and the discharge gas containing the formed compound (5) is condensed and continuously recovered.

The reaction temperature for the gas phase reaction is preferably from 50 to 350°C, particularly preferably from 50 to 300°C, especially preferably from 150 to 250°C. Further, in the gas phase reaction, an inert gas which is inert to the reaction may be present in the reaction system. Such an inert gas may, for example, be nitrogen gas or carbon dioxide gas. In a case where an inert gas is permitted to be present, it is preferably in an amount of from 0.01 to 50 vol% to the compound (4). If the amount of the inert gas added is large, the recovery rate of the product may sometimes decrease.

In a case where the compound (4) is a compound having a high boiling point, it is preferred to carry out the cleavage reaction of the ester bond by a liquid phase reaction by heating it in the form of a liquid in the reactor. The reaction pressure for the liquid phase reaction is not limited. The compound (5) or the compound (6) to be formed by the cleavage reaction of the ester bond usually has a boiling point lower than the compound (4), and it is preferred to carry out the reaction by means of a reaction apparatus having a reflux column and to carry out the reaction while continuously withdrawing the compound (5) from the reaction system by distillation. Further, in such a liquid phase reaction, after the reaction, the product may be withdrawn all at once from the reactor. The reaction temperature for such a liquid phase reaction is preferably from 50 to 300°C, particularly preferably from 100 to 250°C.

The liquid phase reaction may be carried out in the absence of a solvent or in the presence of a solvent (hereinafter referred to as a cleavage reaction solvent). In a case where the reaction is carried out in the absence of a solvent, the compound (4) itself will serve as a solvent. The cleavage reaction solvent is not particularly limited so long as it will not react with the compound (4), and it has compatibility with the compound (4) and will not react with the compounds (5) and (6) to be formed. Further, as such a solvent, it is preferred to select one which can easily be separated at the time of purification of the compound (5). Specific examples of such a cleavage reaction solvent include inert solvents such as a perfluorotrialkylamine and perfluoronaphthalene, and chlorofluorocarbons. Particularly preferred among the chlorofluorocarbons is a chlorotrifluoroethylene oligomer having a high boiling point (such as Flon Lube, trade name). Further, the amount of the cleavage reaction solvent is preferably from 10 to 1000 mass%, based on the compound (4).

Further, in a case where the cleavage reaction of the ester bond is carried out by the reaction with a nucleophilic agent or an electrophilic agent in a liquid phase, the nucleophilic agent is preferably F⁻, particularly preferably F⁻ derived from a fluoride of an alkali metal. As such an alkali metal fluoride, NaF, NaHF₂, KF or CsF is preferred. Among them, NaF is particularly preferred from the viewpoint of economical efficiency.

In a case where a nucleophilic agent (such as F⁻) is employed, F⁻ is nucleophilically added to a carbonyl group present in the ester bond of the compound (4), whereby R^{AF}[OCF(CF₃)CF₂]ₙO⁻ will be detached, and the compound (6) will be formed. From R^{AF}[OCF(CF₃)CF₂]ₙO⁻, F⁻ will further be detached to form the compound (5). The detached F⁻ will likewise react with another molecule of the compound (4). Accordingly, the nucleophilic agent to be used at the initial stage of the reaction may be in a catalytic amount or in an excess amount. Namely, the amount of the nucleophilic agent such as F⁻ is preferably from 1 to 500 mol%, particularly preferably from 1 to 100 mol%, especially preferably from 5 to 50 mol%, based on the compound (4).

In a case where the cleavage reaction of the compound (4) is carried out in the presence of a nucleophilic agent or an electrophilic agent in a liquid phase, it may be carried out in the absence of a solvent or in the presence of a cleavage reaction solvent. The reaction temperature is preferably from -30°C to the boiling point of the solvent or the boiling point of the compound (4), particularly preferably from -20°C to 250°C. In this method, it is preferred to carry out the reaction by means of a reaction apparatus having a reflux column.

In the reaction product of the cleavage reaction of the ester bond, the above-mentioned compound (6) will be present together with the compound (5). In the present invention, only the compound (5) may be obtained from such a reaction product, or the compound (6) may be obtained as well as the compound (5).

In a case where the compound (5) and the compound (6) are different from each other, it is preferred to separate the compound (5) and the compound (6), as the case requires. In a case where the compound (5) and the compound (6) are the same compound, they may be used without separating them. For example, in a case where R^{BF} in the compound (4) is R^{AF}[OCF(CF₃)CF₂]ₙ₋₁OCF(CF₃)-, only the compound (5) will be formed by the cleavage reaction of the ester bond. The compound (5) and/or the compound (6) obtained by the cleavage reaction of the ester bond, may be reacted as the compound (2) with the compound (1). Namely, if it is so designed that a part of the product of the cleavage reaction of the ester bond is recycled to be reacted with the compound (1), the process of the present invention can be carried out as a continuous process simply by supplying the compound (1) to the reaction system.

The following compounds may be mentioned as specific examples of the compound (5). Here, m, n and Cy^{F} are as defined above.

(5A) F (CF₂) ₘ [OCF (CF₃) CF₂] ₙ₋₁ OCF (CF₃) COF

(5B) (CF₃) ₂ CF [OCF (CF₃) CF₂] ₙ₋₁ OCF (CF₃) COF

(5C) (CF₃) ₂ CFCF₂ [OCF (CF₃) CF₂] ₙ₋₁ OCF (CF₃) C OF

(5D) (CF₃) ₃ C [OCF (CF₃) CF₂] ₙ₋₁ OCF (CF₃) COF

(5E) Cy^{F} [OCF (CF₃) CF₂] ₙ₋₁ OCF (CF₃) COF

As specific examples of the compound (6), the same compounds as specific examples of the compound (5) or the compound (2) may be mentioned.

The compound (5) to be produced by the present invention, can be led to a fluororesin monomer by converting the molecular terminal to "-CF=CF₂" by a known pyrolytic reaction (e.g. Methods of Organic Chemistry, 4, Vol. 10b, Part 1, p. 703).

Further, the compound (5) to be produced by the present invention is useful also as a raw material for an emulsifier. Namely, the terminal group -COF of the compound (5) is hydrolyzed to -COOH and then reacted with aqueous ammonia or an alkali metal hydroxide, whereby it can be led to -COO⁻M⁺ (wherein M⁺ is NH₄⁺ or an alkali metal cation).

The hydrolysis can be carried out by a known method. It is preferred to carry out the hydrolysis by using at least 1 time by mol of water to the compound (5). Such a hydrolysis can be carried out also by contacting excess water to the compound (5).

The hydrolysis may be carried out in the presence of an organic solvent. Further, the hydrolysis may be carried out in a homogeneous reaction system or in a heterogeneous system comprising an organic layer and an aqueous layer. If the reaction is carried out in a heterogeneous system, a surfactant, a phase-transfer catalyst or the like may be present in the reaction system. The reaction temperature for the hydrolysis is not particularly limited and is usually preferably from 0 to 50°C. The reaction pressure is not particularly limited and is usually preferably atmospheric pressure. The reaction time is preferably from 0.1 to 5 hours, particularly preferably from 0.5 to 3 hours.

Further, in this hydrolysis, HF will be formed. In order to capture this HF, a basic scavenger such as an alkali metal hydroxide may be added. However, if it is added excessively, there will be a problem such that undesired metal carboxylate will be formed, or an alkali metal fluoride will be formed, and such an undesirable product is likely to be included as an impurity in the final product. Accordingly, in order to remove HF, it is preferred to introduce nitrogen into the reaction system and to remove HF out of the system.

By the hydrolysis, a carboxylic acid compound having the terminal -COF or the compound (5) converted to -COOH, will be formed. In a usual case, it is preferred that after distilling moisture or an acid component off from the reaction crude product containing such a carboxylic acid component, the crude product is used as it is in the subsequent step. If necessary, such a carboxylic acid component may be isolated and purified by a method such as recrystallization, distillation or sublimation.

Further, the carboxylic acid compound thus obtained is reacted with ammonia or an alkali metal hydroxide to form the compound (7). This reaction may be carried out in the presence of an organic solvent. Further, the hydrolysis may be carried out in a homogeneous reaction system or in a heterogeneous system comprising an organic layer and an aqueous layer. In a case where the reaction is carried out in a heterogeneous system, a surfactant, a phase-transfer catalyst or the like, may also be present in the reaction system.

The ammonia or the alkali metal hydroxide is preferably used in the form of its aqueous solution for the reaction with the carboxylic acid compound. The amount of the ammonia or the alkali metal hydroxide is preferably from 1 to 2 times by mol, particularly preferably an equal time by mol to the carboxylic acid compound. Further, in a case where ammonia is used in excess, non-reacted ammonia can easily be removed under reduced pressure, such being preferred. The reaction temperature for the reaction is not particularly limited, and it is usually preferably from 0°C to 50°C. The reaction pressure is not particularly limited, and it is usually preferably atmospheric pressure. The reaction time is preferably from 0.1 to 5 hours, particularly preferably from 0.5 to 3 hours.

From a reaction crude product containing the compound (7) obtained by the reaction with the ammonia or the alkali metal hydroxide, the solvent and excess moisture may be distilled off as the case requires, to obtain the desired compound (7). Further, if necessary, the compound (7) may be purified by a method such as recrystallization or sublimation.

Some of the compounds (7) obtainable by the process of the present invention are novel compounds. Namely, according to the present invention, novel compounds (7A) will be provided.

(7A) R^{AF1} [OCF (CF₃) CF₂] ₙ₋₁ OCF (CF₃) COO⁻M⁺

In the formula, R^{AF1} is a C₄₋₁₀ perfluoroalkyl group, and n is an integer of from 1 to 10. M⁺ is NH₄⁺ or an alkali metal cation.

In the compound (7A), R^{AF1} is preferably a C₆-perfluoroalkyl group (i.e. C₆F₁₃-). Especially from the viewpoint of the performance as an emulsifier, R^{AF1} is particularly preferably F(CF₂)ₘ- (m is an integer of from 4 to 10), especially preferably such a group wherein m is 6 (i.e. F(CF₂)₆-). Further, n is preferably 1.

The compound (7) thus prepared, is useful as a fluorinated surfactant or as an emulsifier.

For example, the compound (7) is useful as an emulsifier in emulsion polymerization, particularly useful as an emulsifier to be used for emulsion polymerization of a fluorinated polymerizable monomer. The fluorinated polymerizable monomer which can be polymerized by emulsion polymerization by means of the compound (7) of the present invention, may, for example, be a fluorinated olefin such as tetrafluoroethylene, hexafluoropropylene, trifluoroethylene, chlorotrifluoroethylene, vinylidene fluoride, vinyl fluoride, perfluoro(methyl vinyl ether), perfluoro(propyl vinyl ether), perfluoro(butenyl vinyl ether) or perfluoro(2,2-dimethyldioxolane) or a fluorinated monomer such as an acrylate or methacrylate having a polyfluoroalkyl group. Such fluorinated polymerizable monomers may be polymerized alone or in combination as a mixture of two or more of them. Or, one or more fluorinated polymerizable monomers may be polymerized with at least one polymerizable monomer containing no fluorine and being polymerizable therewith.

As the method for emulsion polymerization, a known method may be employed. For example, a method may be mentioned wherein the compound (7) of the present invention, the polymerizable monomer and a polymerization initiator are added to an aqueous medium, and polymerization is carried out with stirring. The amount of the compound (7) is preferably from about 0.01 to 2 wt%, based on the aqueous medium. The compound (7) may be added all at once at the initial stage of the polymerization reaction, or a part of the amount is added at the initial stage of the polymerization reaction, and the rest may be added as the polymerization reaction proceeds.

As the polymerization initiator to be used for the emulsion polymerization, it is usually preferred to employ a water-soluble radical generator such as an inorganic peroxide such as ammonium persulfate or potassium persulfate, or an organic peroxide such as disuccinic peroxide. Further; a redox type polymerization initiator by a combination with a reducing agent, may also be employed.

Further, in the emulsion polymerization, other additives may be added as the case requires. As such other additives, a pH-controlling agent, etc. may be mentioned. The polymerization temperature for the emulsion polymerization is usually preferably from 20 to 120°C, particularly preferably from 25 to 80°C. Further, the pressure for the polymerization reaction is preferably at most 3 MPa.

The compound (7) of the present invention has an ether structure at the α-position and thus is a compound which can be present sufficiently stably under the polymerization conditions for the emulsion polymerization. On the other hand, the compound (7) has a nature such that it tends to undergo pyrolysis at a relatively low temperature and thus is a useful compound, of which the influence to the environment such as accumulation, can be minimized. Namely, with the fluororesin emulsion obtained by the emulsion polymerization employing the compound (7) of the present invention, the safety can be improved over the one obtained by using a conventional emulsifier.

According to the process of the present invention, a perfluoroacyl fluoride can be produced in a short process and in high yield by using the compound (1) and the compound (2) which are materials available at low costs. Further, by employing the process of the present invention, a low molecular weight acyl fluoride and a carboxylic acid salt which used to be difficult to obtain by conventional methods, can be produced efficiently. Further, by selecting the structures of R^{A} and R^{B}, the process of the present invention can be made to be a continuous process.

The acyl fluoride and the carboxylic acid salt obtainable by the process of the present invention, are compounds useful as emulsifiers or fluororesin monomers.

### EXAMPLES

Now, the present invention will be described in further detail with reference to Examples. But the present invention is by no means thereby restricted.

In the following, gas chromatography is represented by GC, gas chromatographic mass spectrometry by GC-MS, and infrared absorption spectrum by IR spectrum. Further, the purity determined by the peak area ratio of GC is represented by GC purity, and the yield by GC yield. Further, CCl₂FCClF₂ is represented by R-113.

### Example 1: Example for the preparation of CF₃CF₂CF₂OCF(CF₃)CF₂OCF(CF₃)COF(5a)

The flow chart for the production of the compound (5a) will be shown below. Here, R^{A1} in the following formula represents CH₃CH₂CH₂OCH(CH₃)CH₂-, and R^{AF1} represents -CF(CF₃)OCF₂CF(CF₃)OCF₂CF₂CF₃.

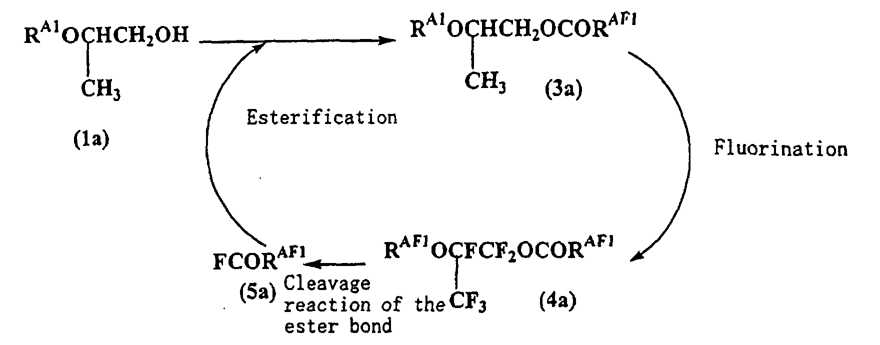

### Example 1-1: Example for the preparation of CH₃CH₂CH₂[OCH(CH₃)CH₂]₂OCO[CF(CF₃)OCF₂]₂CF₂CF₃ (3a) by an esterification reaction

The compound (1a) (88 g) was stirred, and nitrogen gas was bubbled. Then, the compound (5a) (261.5 g) was supplied and reacted while maintaining the reaction temperature at from 20 to 40°C to obtain the compound (3a) (294.3 g). GC-MS of the product: 635 (M⁺-F). Further, as a result of measuring the IR spectrum of the product, a strong absorption attributable to an ester bond was observed at 1780 to 1850 cm⁻¹.

### Example 1-2: Example for the preparation of CF₃CF₂CF₂[OCF(CF₃)CF₂]₂OCO[CF(CF₃)OCF₂]₂CF₂CF₃ (4a) by a fluorination reaction

Into a 500 mL autoclave made of nickel, R-113 (312 g) was added, stirred and maintained at 25°C. A cooler maintained at 20°C, a layer packed with NaF pellets, and a cooler maintained at -10°C were installed in series at the gas outlet of the autoclave. Further, a liquid returning line to return a condensed liquid from the cooler maintained at -10°C to the autoclave, was installed. After supplying nitrogen gas for 1.0 hours, fluorine gas diluted to 20 vol% with nitrogen gas (hereinafter referred to as diluted fluorine gas) was supplied for one hour at a flow rate of 0.75 L/hr. Then, while supplying diluted fluorine gas at the same flow rate, a solution having the compound (3a) (8 g) obtained in Example 1-1 dissolved in R-113 (160 g), was injected over a period of 4.8 hours.

Then, while supplying diluted fluorine gas at the same flow rate, a R-113 solution containing benzene at a concentration of 0.01 g/ml, was injected in an amount of 9 mL while raising the temperature from 25°C to 40°C, whereupon the benzene inlet of the autoclave was closed, and further, the outlet valve of the autoclave was closed. When the pressure became 0.20 MPa, the inlet valve for fluorine gas of the autoclave was closed, and stirring was continued for 0.8 hour. Then, the pressure was returned to atmospheric pressure, and while maintaining the internal temperature of the reactor at 40°C, 6 mL of the above benzene solution was injected, whereupon the benzene inlet of the autoclave was closed, and further the outlet valve of the autoclave was closed. When the pressure became 0.20 MPa, the inlet valve for fluorine gas of the autoclave was closed, and stirring was continued for 0.5 hour. Further, the same operation was repeated three times. The total amount of benzene injected was 0.451 g, and the total amount of R-113 injected was 45 mL.

Further, nitrogen gas was supplied for 1.5 hours to obtain the compound (4a). GC-MS of the product: 977 (M⁺-F). Further, as a result of measuring the IR spectrum, a strong absorption attributable to an ester bond was observed at 1780 to 1850 cm⁻¹.

### Example 1-3: Example for the preparation of CF₃CF₂CF₂OCF(CF₃)CF₂OCF(CF₃)COF (5a) by a liquid phase ester cleavage reaction

The compound (4a) (8.5 g) obtained in Example 1-2 was charged together with NaF powder (0.1 g) into a flask and heated for 9.5 hours at 190°C in an oil bath, while stirring vigorously. At an upper portion of the flask, a reflux condenser adjusted to a temperature of 20°C was installed. After cooling, 8.3 g of a liquid sample was recovered. As a result of an analysis by GC-MS, the compound (5a) was confirmed to be the main product. Further, in the IR spectrum of the main product, an absorption attributable to -COF was observed in the vicinity of 1880 cm⁻¹. the GC yield was 85%.

### Example 1-4: Example for the preparation of CF₃CF₂CF₂OCF(CF₃)CF₂OCF(CF₃)COO⁻NH₄⁺

Into a 30 L plastic container, the compound (5a) (6662.9 g) obtained in the method of Example 1-3, as diluted with R-225, was added, and pure water (234.8 g) was dropwise added while stirring vigorously. While removing HF formed, by nitrogen blowing, stirring was continued for 22 hours, and the reaction was terminated at a stage where disappearance of the compound (5a) was confirmed by GC. By bubbling nitrogen for further 5 hours with stirring, HF was removed until the F⁻ concentration became 15 ppm, to obtain CF₃CF₂CF₂OCF(CF₃)CF₂OCF(CF₃)COOH.

The solution was transferred to a 20 L round bottomed flask, and then NH₃ gas was fed at a flow rate of 80 NL/hr while stirring vigorously under cooling with ice bath for conversion to an ammonium salt. After confirming the neutralization by measuring the pH, nitrogen bubbling was carried out to remove excess ammonia. The obtained solution was evaporated to remove excess water azeotropically with R-225, to obtain a highly viscous ammonium salt solution. Thereafter, R-225 was added again and dissolved and left to stand still under cooling at -1 to 3°C to carry out purification by reprecipitation. The obtained white gelled precipitate was subjected to evaporation to distill off R-225. As a result, the above identified compound was obtained in an amount of 5189.7 g (yield: 75.6%) as slightly viscous white solid. The concentration of R-225 remaining in this white solid was less than 100 ppb.

### Example 2: Example for the preparation of F(CF₂)₆OCF(CF₃)COF (5b)

An esterification reaction was carried out in the same manner as in Example 1-1 by changing the compound (1a) to H(CH₂)₆OCH(CH₃)CH₂OH (1b) and changing the compound (5a) to FCOCF(CF₃)O(CF₂)₆CF₃ (5b), to obtain H(CH₂)₆OCH(CH₃)CH₂OCOCF(CF₃)O(CF₂)₆CF₃ (3b). Further, a liquid phase fluorination reaction was carried out in the same manner as in Example 1-2 by changing the compound (3a) to the compound (3b) obtained by the above reaction, to obtain F(CF₂)₆OCF(CF₃)CF₂OCOCF(CF₃)O(CF₂)₆CF₃ (4b). Further, a cleavage reaction of an ester bond was carried out in the same manner as in Example 1-3 by changing the compound (4a) to the compound (4b) obtained by the above reaction, to obtain the above-identified compound (5b).

### Example 3: Example for the preparation of F(CF₂)₆OCF(CF₃)COO⁻NH₄⁺ (7a)

Into a 100 mL flask, F(CF₂)₆OCF(CF₃)COF (2.0 g) obtained in Example 2 and deionized water (30 mL) were added. While removing HF generated, by nitrogen blowing, stirring was continued for 3 hours. At the initial stage of the reaction, the reaction system was separated in two layers, but as the time passed, the reaction system became one layer. When it became one layer, the reaction was terminated. Then, water and the HF content were distilled off under reduced pressure, to obtain F(CF₂)₆OCF(CF₃)COOH as a viscous liquid.

Further, 20 mL of deionized water was added to F(CF₂)₆OCF(CF₃)COOH, followed by stirring, whereupon 0.1 mol/L of aqueous ammonia was added, followed by stirring for one hour for conversion to an ammonium salt. After neutralization was confirmed by measuring the pH, the obtained aqueous solution was freeze-dried to remove moisture and excess ammonia thereby to obtain F(CF₂)₆OCF(CF₃)COO⁻NH₄⁺ (white solid, 1.6 g, yield: 77.5%). This white solid was measured by thermogravimetry, whereby the decomposition temperature was such that the 50% decomposition temperature was 180°C, and the 90% decomposition temperature was 199°C.

The ¹⁹F⁻NMR spectrum and the ¹H-NMR spectrum of the product are shown below.
¹⁹F-NMR (376.0 MHz, solvent: D₂O, standard: CFCl₃) δ (ppm):-83.6(2F), -83.7(3F), -84.0(3F), -123.9(2F), -124.6(2F), -127.2(2F), -127.4(1F), -128.2(2F)
¹H-NMR (399.0 MHz, solvent: D₂O, standard: TMS) δ (ppm): 7.2 (4H)

### Reference Example 1: Example for the preparation of polytetrafluoroethylene (Part 1)

Into a 100 L polymerization vessel, paraffin wax (736 g), ultra pure water (59 L), the compound (7a) (36.8 g) prepared in Example 3 were charged. The temperature was raised to 70°C, and deaeration and nitrogen purging were carried out. Then, tetrafluoroethylene (hereinafter referred to as TFE) was introduced, and the pressure was adjusted to 1.9 MPa. With stirring, 1 L of 0.5 mass% aqueous solution of disuccinic peroxide was injected to initiate the polymerization. As the polymerization proceeded, TFE was consumed, and the pressure in the polymerization vessel decreased. To maintain the pressure to be constant, TFE was continuously supplied during the polymerization. After expiration of 45 minutes from the initiation of the polymerization, the temperature was raised to 90°C at a rate of 6°C/hr. Further, when the amount of TFE supplied became 6.6 kg, the compound (7a) obtained in Example 3 (1 L of a 6.2 mass% aqueous solution) was added.

Upon expiration of 160 minutes from the initiation of the polymerization, stirring and supply of TFE were stopped, and TFE in the polymerization vessel was purged to terminate the polymerization. The obtained polytetrafluoroethylene (hereinafter referred to as PTFE) aqueous dispersion having a solid content of 24.7 mass%, was coagulated, and PTFE in a wet state, was separated. The obtained PTFE in a wet state, was dried at 205°C to obtain a PTFE fine powder. The standard specific gravity of the obtained polymer was 2.156, and the average particle size of agglomerated particles was 491 µm.

### Reference Example 2: Example for the preparation of PTFE (Part 2)

Into a 100 L polymerization vessel, paraffin wax (928 g), ultra pure water (55 L), the compound (7a) (40.2 g) obtained in Example 3, succinic acid (1 g) and a 1 mol/L nitric acid aqueous solution (8 mL) and potassium hydrogen bromide (0.4 g) were charged. After carrying out deaeration and nitrogen purging, the temperature was raised to 85°C. After stabilization of the temperature, TFE was introduced, and the pressure was adjusted to 1.9 MPa. While stirring the content, ammonium sulfite (1 L of a 140 ppm aqueous solution) was continuously added for 60 minutes to initiate the polymerization. As the polymerization proceeded, TFE was consumed, and the pressure in the polymerization vessel decreased. Accordingly, in order to maintain the pressure to be constant, TFE was supplied continuously during the polymerization. After completion of the addition of ammonium sulfite, the compound (7a) obtained in Example 3 (1 L of a 12.4 mass% aqueous solution) was added.

Upon expiration of 270 minutes from the initiation of the polymerization, stirring and supply of TFE were stopped, and TFE in the polymerization vessel was purged. Then, the gas phase was substituted by nitrogen. The obtained PTFE aqueous dispersion having a solid content of 29.6 mass%, was coagulated in the presence of ammonium carbonate, and PTFE in a wet state was separated. The obtained PTFE in a wet state was dried at 250°C to obtain PTFE fine powder. The same post treatment as in Reference Example 1 was carried out. The obtained PTFE fine powder had a standard specific gravity of 2.149, and the average particle size of agglomerated particles was 469 µm.

### Reference Example 3: Example for the preparation of a copolymer of tetrafluoroethylene and propylene

Into a 1 L polymerization vessel, deionized water (550 g), 2-propanol (64 g), the compound (7a) (4.5 g) obtained in Example 3, disodium hydrogen phosphate 12 hydrate (19 g), sodium hydroxide (2 g), ammonium persulfate (6 g), ethylene diamine tetraacetate (EDTA) (0.04 g) and ferrous sulfate heptahydrate (0.03 g) were charged. The polymerization vessel was flushed with nitrogen and then deaerated, and the temperature was raised to 25°C. Then, a mixed gas of TFE/propylene=90/10 (mol%) was charged to the polymerization vessel until the pressure became 2.45 MPa. On the other hand, a sodium formaldehyde sulfoxylate/sodium hydroxide mixed aqueous solution wherein the concentration of sodium formaldehyde sulfoxylate was 20%, and the concentration of sodium hydroxide was 2.4%, was continuously added to the polymerization vessel to initiate the polymerization. As the polymerization reaction started, the polymerization pressure decreased, and in order to supplement this decrease, a mixed gas of TFE/propylene = 56/44 (mol%) was continuously supplied.

The addition rate of the sodium formaldehyde sulfoxylate/sodium hydroxide mixed aqueous solution was such that the addition was continued for 10 hours to maintain a polymerization rate of about 20 g/hr. Thereafter, addition of the sodium formaldehyde sulfoxylate/sodium hydroxide aqueous solution was terminated, and the polymerization was finally terminated when 290 g was supplied. After completion of the polymerization, the polymerization vessel was cooled, and nitrogen substitution was carried out. The solid content concentration of the obtained latex was 33.8%. This latex was salted out and coagulated with calcium chloride, washed and dried to obtain a polymer.

The obtained polymer was a polymer wherein from the analysis of the fluorine content, the composition of TFE/propylene was 56.1/43.9 mol%, and from the thermal analysis, the glass transition temperature was 2°C. Further, the Mooney viscosity of this polymer was measured in accordance with the method for the Mooney viscosity test as described in JIS K6300, whereby it was 92.2 ML (1+4) 100°C, and 93.6 ML (1+10) 100°C.

### INDUSTRIAL APPLICABILITY

According to the present invention, it is possible to produce an acyl fluoride and a carboxylic acid salt useful as a raw material for a fluororesin monomer or an emulsifier, with efficient productivity while preventing side reactions.

The process of the present invention can be carried out without using CsF and thus is a process which is economically more advantageous than a conventional process. Further, according to the process of the present invention, as is different from the process for production by an addition reaction, a product corresponding to the structure of the starting material can be produced, and it is possible to obtain a product having the carbon number of the group controlled. Further, it is possible to obtain a desired compound containing little impurities.

Further, by the production by the process of the present invention, an emulsifier having a desired carbon number can be produced. Further, production of emulsifiers having various structures is easy. Namely, according to the present invention, it is possible to produce an emulsifier of a structure which corresponds to the desired performance. Accordingly, various emulsifiers will be available which satisfy the stability of the emulsifiers and the required performance, etc. when used for polymerization reactions.

## Claims

1. A process for producing a compound of the following formula (5), or a compound of the following formula (5) and a compound of the following formula (6), which comprises reacting a compound of the following formula (1) and a compound of the following formula (2) to form a compound of the following formula (3), fluorinating in a liquid phase the compound of the formula (3) thus formed, to obtain a compound of the following formula (4), and then cleaving an ester bond in the compound of the formula (4):
(1) R^{A} [OCH(CH₃)CH₂] ₙ OH
(2) XCOR^{B}
(3) R^{A} [OCH(CH₃)CH₂] ₙ OCOR^{B}
(4) R^{AF} [OCF(CF₃)CF₂] ₙ OCOR^{BF}
(5) R^{AF} [OCF(CF₃)CF₂] ₙ₋₁ OCF(CF₃)COF
(6) FCOR^{BF}
wherein R^{A} is a group which becomes R^{AF} upon fluorination, R^{AF} is a perfluoroalkyl group, R^{B} is the same monovalent organic group as R^{BF} or a monovalent organic group which becomes R^{BF} upon fluorination, R^{BF} is a monovalent organic group, X is a halogen atom, and n is an integer of from 1 to 10.

2. The process according to Claim 1, wherein each of R^{B} and R^{BF} is R^{AF}[OCF(CF₃)CF₂]ₙ₋₁OCF(CF₃)-, wherein n is as defined above.

3. The process according to Claim 1 or 2, wherein n is 2.

4. A process for producing a compound of the following formula (7), which comprises hydrolyzing the compound of the formula (5) obtained by the process of any one of Claims 1 to 3, followed by a reaction with ammonia or an alkali metal hydroxide:
(7) R^{AF} [OCF(CF₃)CF₂]ₙ₋₁OCF (CF₃)COO⁻M⁺
wherein R^{AF} and n are as defined above, M⁺ is NH₄⁺ or an alkali metal cation.

5. A compound of the following formula (4A): 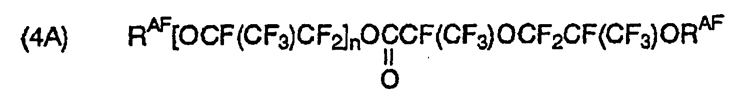 wherein R^{AF} is a perfluoroalkyl group, and n is an integer of from 1 to 10.

6. A compound of the following formula (7A):
(7A) R^{AF1} [OCF (CF₃) CF₂] ₙ₋₁ OCF (CF₃) COO⁻M⁺
wherein R^{AF1} is a C₄₋₁₀ perfluoroalkyl group, n is an integer of from 1 to 10, and M⁺ is NH₄⁺ or an alkali metal cation.

7. The compound of the formula (7A) according to Claim 6, wherein n is 1.

8. The compound of the formula (7A) according to Claim 6 or 7, wherein R^{AF1} is C₆F₁₃-.

9. An emulsifier comprising the compound of the formula (7A) as defined in any one of Claims 6 to 8.
